# EUROPÄISCHE PATENTSCHRIFT

(11) **EP 1 917 982 B1**
(45) Veröffentlichungstag und Bekanntmachung des Hinweises auf die Patenterteilung: **21.04.2010**
(21) Anmeldenummer: 06023033.1
(22) Anmeldetag: 06.11.2006
(51) Int. Cl.: A61L 15/32, A61K 9/00

(54) **Produkt zur Versorgung von Entzündungen, Druckstellen und/oder Aphten im Oralbereich sowie Verwendung eines solchen Produkts**
Product for the treatment of inflammation, bruises and/or ulcers in the oral area and the use of such a product
Produit de traitement de l'inflammation, les contusions et/ou les aphtes pour les soins buccaux et utilisation d'un tel produit

(43) Veröffentlichungstag der Anmeldung: 07.05.2008
(60) Teilanmeldung: 10000324.3
(73) Patentinhaber: Lohmann & Rauscher GmbH & Co. KG, 56567 Neuwied (DE)
(72) Erfinder: Scholz, Gert Hartmut Dr., 56567 Neuwied (DE)
(74) Vertreter: Leinweber & Zimmermann

(56) Entgegenhaltungen:
- EP-A- 1 795 210
- EP-A1- 1 252 903
- WO-A-2004/043351
- DE-A1- 3 832 162
- US-A- 4 614 794

## Beschreibung

Die Erfindung betrifft ein Produkt zur Versorgung von Entzündungen, Druckstellen und/oder Aphthen im Oralbereich aus einem überwiegend, vorzugsweise vollständig, bioresorbierbaren Material sowie die Verwendung eines derartigen Produktes.

Beschwerden im Oralbereich können unterschiedliche Ursachen haben. Beispielsweise ist die Mundschleimhaut dann anfälliger, wenn sie zu trocken ist. Das kommt manchmal mit steigendem Alter vor, kann aber auch als Nebenwirkung von bestimmten Medikamenten auftreten. Auch bei einem geschwächten Immunsystem können Entzündungen der Mundschleimhaut auftreten. Eine andere Ursache für Probleme im Oralbereich können Vitaminmangel oder allergische Reaktionen sein. Dabei können häufige Entzündungen der Mundschleimhaut auf einen Mangel an bestimmten Vitaminen oder Eisen hindeuten. Auch allergische Reaktionen auf Speisen oder Medikamente können die Ursache sein.

Besonders häufig werden Probleme im Oralbereich durch Zahnprothesen verursacht. Dabei gibt es im besonderen bei der Neuanpassung von Teil- oder Vollprothesen im Ober- und Unterkieferbereich oft lange Eingewöhnungszeiten von mehreren Wochen, während denen Schmerzen und Druckstellen auftreten können. Ähnliche Probleme treten bei Zahnspangen auf. Diese können an der Mundschleimhaut regelrecht scheuern und dadurch Druckstellen und Entzündungen verursachen. Auch bei zahnärztlichen Eingriffen sind Druckstellen durch Instrumente manchmal kaum zu vermeiden. Oft entstehen nach einer Behandlung an solchen Stellen erst nach Stunden oder wenigen Tagen kleine Wunden. Verletzungen im Mundraum können auch durch den Einsatz von Zahnstochern, Gabeln, Knochen, Gräten oder aber durch einen abgebrochenen, scharfkantigen Zahn auftreten. Es kommt ebenfalls nicht selten vor, daß man sich versehentlich auf die Wangen, die Lippen oder die Zunge beißt. Manchmal ist es sogar eine zu harte Zahnbürste, die beim Zähneputzen das Zahnfleisch oft zunächst erst unbemerkt verletzt. Ferner gibt es Mundschleimhautentzündungen, deren Ursache nicht eindeutig feststellbar ist. Ursache davon können beispielsweise Pilzinfektionen (Mundsoor), Virusinfektionen (z.B. mit Herpesviren) oder durch Bakterien verursachte Infektionen sein.

Eine der häufigsten Krankheiten der Mundschleimhaut sind die sog. Aphthen. Aphthen sind meist linsengroße, schmerzhafte Schleimhautveränderungen, die von einem roten entzündlichen Hof umgeben sind. Am häufigsten treten sie am Mundboden, an der Wangen- und Zungenschleimhaut und an der Innenseite der Lippen auf. Manchmal werden sie durch Verletzungen ausgelöst, wie sie oben beschrieben wurden, oder sie treten einfach als unangenehme Begleiterscheinung von Infektionskrankheiten auf. Die eigentliche Ursache konnte die Wissenschaft jedoch bis heute nicht feststellen.

Zur Behandlung der gerade beschriebenen Entzündungen, Druckstellen und/oder Aphthen im Oralbereich sind zahlreiche Präparate bekannt. Diese Präparate liegen in Form von Lösungen zum Spülen, Gelen, Sprays, Pastillen oder Lutschtabletten vor. Sie enthalten Wirkstoffe wie Benzalkonium oder Cetrimonium, Chlorhexidin, Cetylpyridiniumchlorid, Cresol, Dichlorbenzylalkohol oder Hexetidin. Der Wirkstoff Tyrothricin ist ein Antibiotikum, das die entzündungsauslösenden Bakterien unschädlich macht. Ferner sind einige pflanzliche Mittel bekannt, die desinfizierend wirken und Entzündungen hemmen. Auch hier gibt es zahlreiche Darreichungsformen, z.B. Salben, Gele, Sprays, Lösungen und Tinkturen. Sie enthalten beispielsweise Auszüge von Heilpflanzen wie Salbei, Kamille, Pfefferminze, Echinacea (Sonnenhut), Nelkenbaum, Myrrhenstrauch und Rhabarber. Pfefferminz- und Nelkenöl zeigen neben antiseptischen auch örtlich betäubende Effekte. Echinaceaextrakte aktivieren das körpereigene Abwehrsystem. Myrrhetinkturen und Rhabarberwurzelextrakte wirken adstringierend und schützen dadurch die Schleimhäute.

Mit den bekannten Präparaten zur Versorgung von Entzündungen, Druckstellen und/oder Aphthen werden beachtliche Erfolge erzielt. Diese Präparate müssen allerdings oft in hoher Dosierung verwendet werden. Ferner kommt es in vielen Fällen, insbes. bei Problemen im Bereich von Prothesen oder Zahnspangen, bisweilen kurz nach Absetzung der Präparate zu erneuten Entzündungen und/oder Druckstellen. Weitere Produkte zur Versorgung von Entzündungen im Oralbereich sind in der WO2004/043351 und der EP-A1-1252903 beschrieben.

Angesichts der genannten Probleme im Stand der Technik liegt der Erfindung die Aufgabe zugrunde, Produkte zur Versorgung von Entzündungen, Druckstellen und/oder Aphthen bereitzustellen, mit denen bei geringer Dosierung nachhaltig Heilungserfolge erzielt werden können.

Erfindungsgemäß wird diese Aufgabe durch die im kennzeichnenden Teil des Patentanspruchs 1 angegebene Weiterbildung der bekannten Produkte gelöst.

Diese Erfindung geht auf die Erkenntnis zurück, daß die Probleme im Stand der Technik hinsichtlich der zum Erhalt des gewünschten Heilungserfolgs erforderlichen hohen Dosierung im wesentlichen darauf zurückgehen, daß sich die üblicherweise eingesetzten Lösungen, Gele oder Sprays im gesamten Mundraum verteilen, so daß zur Behandlung lokaler Entzündungen oder Druckstellen große Mengen entsprechender Mittel benutzt werden müssen. Dieses Problem kann beseitigt werden, wenn das zur Versorgung von Entzündungen, Druckstellen und/oder Aphthen eingesetzte bioresorbierbare Material ein an der Schleimhaut haftendes Polsterelement aufweist. Das bioresorbierbare Material kann dann gezielt an die problematische Stelle im Mundraum gebracht werden. Eine übermäßig hohe Wirkstoffmenge ist bei Einsatz erfindungsgemäßer Produkte nicht mehr erforderlich.

Gemäß einem weiteren Gesichtspunkt der Erfindung ist das bioresorbierbare Material als flexibles und/oder kompressibles Polstetelement ausgeführt. Dieser Gesichtspunkt der Erfindung geht auf die Erkenntnis zurück, daß das rasche Wiederauftreten von Problemen im Bereich von Voll prothesen und/oder Zahnspangen durch den von diesen Elemen-ten ausgeübten lokalen Druck verursacht wird. Dieser lokale Druck kann mit Hilfe erfindungsgemäß ausgeführter flexibler und/oder kompressibler Polsterelemente über größere Bereiche verteilt bzw. gedämpft werden. Dadurch kann die Ursache für Druckstellen und Entzündungen mit Hilfe erfindungsgemäßer Produkte beseitigt werden.

Im Hinblick auf die gewünschte Bioresorbierbarkeit bei gleichzeitiger Sicherstellung flexibler und/oder kompressibler Eigenschaften ist erfindungsgemäß vorgesehen, daß das bioresorbierbare Material ein vorzugsweise mit mindestens einem Begleitstoff versetztes Kollagen und ein Alginat, insbesondere Ca-Alginat, als weiteres bioresorbierbares Polymer, aufweist. Kollagen kann in Form von kompressiblen Schwämmen bereitgestellt werden, die bei Befeuchten an der Mundschleimhaut haften, im trockenen Zustand aber keine haftenden Eigenschaften aufweisen. Das macht die Lagerung entsprechender Produkte einfach und sichert gleichzeitig die erfindungsgemäß gewünschten Wirkungen hinsichtlich der Haftungseigenschaften an der Schleimhaut, der Flexibilität und/oder der Kompressibilität. Verfahren zum Herstellen von zur Herstellung erfindungsgemäßer Produkte einsetzbaren Kollagenschäumen sind beispielsweise in der DE 38 32 162 C2 beschrieben. Gemäß dieser Schrift können im Rahmen der Erfindung einsetzbare Kollagenschäume hergestellt werden, indem ein gelöstes oder dispergiertes Kollagen in Scheibenform gegossen wird, die Kollagenlösung oder -dispersion in der Scheibenform eingefroren und anschließend gefriergetrocknet wird, eine oder mehrere Kollagenschaumscheiben nebeneinander vertikal auf einen Wickelkern geschoben und maschinell aus den gefriergetrockneten Scheiben des Kollagens durch einen Schälvorgang Endlosbänder hergestellt und diese Bänder zu Rollen aufgewickelt werden. Das Kollagen kann aus junger Kalbshaut stammen, die zunächst enthaart und entfettet wird. Danach werden lösliche Kollagene und die nicht kollagenen Bestandteile des Bindegewebes extrahiert. Bei dem zurückbleibenden, in Wasser unlöslichen Kollagen werden die Telopeptide, die für die antigenen Eigenschaften des Kollagens verantwortlich sind, abgespalten und durch ein entsprechend gesteuertes Verfahren ein definierter Teil der intermolekularen Kollagenbindungen gespalten. Die intramolekularen Bindungen des Kollagens werden nicht angegriffen, so daß die helikale Struktur des Moleküls intakt bleibt. Die Spaltung kann so geführt werden, daß ein Großteil des Kollagens wasserlöslich gemacht wird, so daß nach Filtration oder Zentrifugation eine von unlöslichem Kollagen befreite Lösung erhalten wird. Wird die Spaltung so gesteuert, daß nur eine geringe Anzahl intermolekularer Bindungen gespalten wird, werden überwiegend höhermolekulare wasserunlösliche Kollagenaggregate erhalten, die in Wasser dispergiert werden. Eine stabile Lösung enthält bis zu etwa 5 Gew.-%, vorzugsweise bis zu 2 Gew.-% Kollagen in gleichmäßiger Verteilung. Zur Herstellung von gefriergetrockneten Kollagenschäumen mit hoher mechanischer Festigkeit werden vorzugsweise Dispersionen mit sehr hohem Anteil an hochmolekularen Aggregaten verwendet, da durch den hohen natürlichen Vernetzungsgrad des Kollagens der Schaum stabilisiert wird. In die Kollagenzubereitung können weitere Stoffe eingearbeitet werden. Dabei kann es sich bei dem erfindungsgemäß einsetzbaren Kollagen um marktübliches bovines, equines, porkines und/oder marines Kollagen handeln. Zum Erhalt gewünschter Eigenschaften, wie etwa zum Erhalt einer gewünschten Flexibilität und Kompressibilität, kann das bioresorbierbare Material zusätzlich zu Kollagen und dem Alginat noch mindestens ein weiteres bioresorbierbares Polymer aufweisen. Dabei kann das weitere bioresorbierbare Polymer Gellan, Chitosan, Chitin, κ-Karagenan, ϕ-Karagenan, Xanthan, Carboxymethylcellulose, Hydroxyethylcellulose und/oder Hydroxyethylpropylcellulose aufweisen.

Das erfindungsgemäß eingesetzte Alginat beeinflußt die Freisetzung von polykationischen Wirkstoffen (z.B. Biguanide wie Polyhexanid). Dabei kann von einer kontinuierlichen Wirkstoffabgabe ausgegangen werden. Das Ca-Alginat beeinflußt die mechanische Belastbarkeit und Biodegradierbarkeit. Ferner ist bei Einsatz von Ca-Alginat ein positiver Nutzen in der Wundheilung beschrieben. Besonders hervorzuheben ist, daß durch die Zugabe von Ca-Alginat zu Kollagen eine besonders hohe reversible Kompressibilität des bioresorbierbaren Materials erreicht werden kann.

Gellan kann die Freisetzung von Biguaniden ebenfalls beeinflussen. Dazu ist in der Literatur im Hinblick auf Gellan ebenfalls eine wundheilungsfördernde Wirkung beschrieben.

Chitosan besitzt einen bakteriostatischen Effekt. Außerdem besitzt Chitosan einen hämostatischen Effekt. Dieser wirkt sich auch auf die Kombination mit der bioresorbierbaren Matrix aus. Es kommt zu einer Erhöhung der hämostatischen und der bakteriostatischen Wirkung. Ferner stellt Chitosan einen schlechten Nährboden für Mikroorganismen dar. Daher verhindert der Einsatz von Chitosan ein schnelles Verkeimen der Kollagenauflage, die von Natur aus einen guten Nährboden für Mikroorganismen darstellt. In einer besonderen Ausführungsform wird Chitosan in einer löslichen Form zugegeben, so daß es aus der Kollagenmatrix austritt und durch seine filmbildenden Eigenschaften das Mikroklima im Mundraum positiv beeinflußt. Chitin ist in Wasser nicht löslich. Daher wird Chitin zweckmäßigerweise in Form eines Copolymers von Chitosan und Chitin eingesetzt. Dieses Copolymer hat etwa die gleichen Eigenschaften wie Chitosan.

In der Kombination mit Biguaniden, wie z.B. Polyhexanid, kommt es durch eine Veränderung der ionischen Ladung der Kollagenmatrix zu einer erhöhten Freisetzung des Wirkstoffs.

κ-Karagenan ist biodegradierbar und besitzt nur eine geringe Löslichkeit. Daher kann durch Zugabe von κ-Karagenan die Kollagenmatrix hydrophober eingestellt werden.

ϕ-Karagenan besitzt eine bessere Löslichkeit. Es fungiert insbes. in der durchspeichelten Probe zur Verbesserung des Mundgefühls.

Xanthan ist ein sehr effektives Verdickungsmittel. Dies führt ebenfalls zu einer Verbesserung des Mundgefühls, insbes. in einer durchspeichelten Kollagenmatrix.

Die Zugabe von Carboxymethylcellulose führt ebenfalls zu einer Verbesserung des Mundgefühls. Aufgrund der polyanionischen Natur der Komponente wird die Abgabe von Biguaniden herabgesetzt. Es wird ein Verkeimen der Auflage verhindert. Die Wirkstoffabgabe (z.B. Polyhexanid) ist äußerst gering.

Hydroxyethylcellulose und Hydroxyethylpropylcellulose führen in Kombination mit Kollagen zu einer Verbesserung des Mundgefühls.

Die Konzentration des als weiteres bioresorbierbares Material eingesetzten Alginats beträgt vorzugsweise 0,5 bis 50 %, bezogen auf das Trockengewicht des bioresorbierbaren Trägermaterials (Kollagen). Bei einer Konzentration von weniger als 0,5 % werden die angestrebten Wirkungen nicht erhalten. Bei einer Konzentration von 50 % oder mehr werden die Eigenschaften des Trägermaterials nachteilhaft beeinflußt. Beispielsweise besteht die Gefahr, daß die gewünschte Polsterwirkung dann nicht mehr erreicht wird. Das gilt allerdings nicht für Honig. Dieser kann auch in höheren Konzentrationen zugesetzt werden, ohne die Eigenschaften des Trägermaterials negativ zu beeinflussen.

Gemäß einer weiteren Ausführungsform der Erfindung weist das bioresorbierbare Material mindestens einen die Erhöhung der Haftung an der Schleimhaut, die Verbesserung der Flexibilität bzw. Anschmiegsamkeit und/oder die Verbesserung des Geschmacks bewirkenden Begleitstoff auf. Dieser Begleitstoff kann Honig, Propolis, Aloe Vera, Panthenol, Polyethylenglykol 200-2000, Polyvinylpyrrolidon, Glycerin, Kamilleextrakt, Salbeiöl, Pfefferminzöl, Vitamine und/oder Provitamine aufweisen. Honig kann in einer Konzentration von 60 % oder mehr, bezogen auf den Trockengehalt der Kollagenmatrix, zugesetzt werden. Glycerin kann in einer Menge von 0,1 bis 50 %, bezogen auf den Trockengehalt der Kollagenmatrix, zugesetzt werden. Der Kamilleextrakt wird vorzugsweise in einer Menge von 0,5 bis 1,5 %, insbes. etwa 1 %, bezogen auf den Trockengehalt der Kollagenmatrix, hinzugesetzt. Ebenso wird das Salbeiöl vorzugsweise in einer Menge von 0,5 bis 1,5 %, insbes. etwa 1 %, bezogen auf den Trockengehalt der Kollagenmatrix, hinzugesetzt. Vitamin C kann ebenfalls in einer Menge von 0,5 bis 1,5 %, bezogen auf den Trockengehalt der Kollagenmatrix, insbes. in einer Menge von etwa 1 %, beigefügt werden. Zitronenextrakt wird ebenfalls in einer Menge von 0,5 bis 1,5 %, bezogen auf den Trockengehalt der Kollagenmatrix, insbes. in einer Menge von etwa 1 %, beigefügt.

Die bislang genannten Begleitstoffe Honig, Glycerin, Kamilleextrakt, Salbeiöl, Pfefferminzöl und Vitamin C sowie Zitronenextrakt, Cyclamat und andere synthetische Süßstoffe dienen ebenso wie der ebenfalls als Begleitstoff einsetzbare Orangenextrakt zur Geschmacksverbesserung. Dabei werden Cyclamat und andere synthetische Süßstoffe vorzugsweise in einer Menge von 0,2 bis 0,8 %, insbes. etwa 0,5 %, bezogen auf den Trokkengehalt der Kollagenmatrix, eingesetzt, während Orangenextrakt in einer Menge von 0,5 bis 1,5 %, insbes. etwa 1 %, bezogen auf den Trockengehalt der Kollagenmatrix, beigefügt wird. Minzöle, insbes. Pfefferminze (*Mentha x piperita*), wird zweckmäßigerweise in einer Menge von 0,5 bis 1,5 % eingesetzt.

Eine Geruchsverbesserung kann durch Zugabe von Propolis in einer Menge von 0,5 bis 1,5 %, insbes. etwa 1 %, bezogen auf den Trockengehalt der Kollagenmatrix ebenso erzielt werden wie durch Zugabe von Extrakten von Zitrusfrüchten in einer Menge von 0,5 bis 1,5 %, insbes. etwa 1 %, bezogen auf den Trockengehalt der Kollagenmatrix.

Eine haftungsverändernde Wirkung wird durch Glycerin, Panthenol, Polyethylenglykol 200-2000, Polyvinylpyrrolidon, Aloe Vera oder auch durch andere biodegradierbare Polymere erzielt. Dabei beträgt die Glycerinmenge vorzugsweise etwa 5 bis 50 %, bezogen auf den Trockengehalt der Kollagenmatrix. Panthenol wird zweckmäßigerweise in einer Menge von 2 bis 5 %, bezogen auf den Trockengehalt der Kollagenmatrix, beigefügt. Polyethylenglykol 200-2000 kann in einer Menge von 1 bis 5 %, insbes. etwa 3 %, bezogen auf den Trockengehalt der Kollagenmatrix, eingesetzt werden. Polyvinylpyrrolidon wird zweckmäßigerweise in einer Menge von 1,5 bis 4,5 %, insbes. etwa 3 %, bezogen auf den Trokkengehalt der Kollagenmatrix, eingesetzt. Die Aloe Vera Menge beträgt vorzugsweise etwa 1 bis 3 %, insbes. etwa 2 %, bezogen auf den Trockengehalt der Kollagenmatrix.

Insgesamt ist es zweckmäßig, wenn die Konzentration der Begleitstoffe im Bereich von 0,5 bis 50 %, bezogen auf das Trockengewicht des Trägermaterials (Kollagen), liegt. Bei einer Konzentration von weniger als 0,5 % werden die gewünschten Wirkungen üblicherweise nicht erreicht, während bei einer Konzentration von mehr als 50 % die Eigenschaften der Kollagenmatrix nachteilhaft beeinflußt werden können.

Bei einer weiteren Ausführungsform der Erfindung kann der zum Versetzen des Kollagens benutzte Begleitstoff ein Anästhetikum und/oder Antiseptikum aufweisen, wobei das Antiseptikum bzw. Lokalanästhetikum aus der Gruppe der Ester bzw. Säureamide der Paraaminobenzoesäure ausgewählt sein kann. Dem Antiseptikum bzw. Lokalanästhetikum kann eine geringe Menge Adrenalin zur Wirkstoffverbesserung zugesetzt sein. Zweckmäßigerweise weist das Anästhetikum Ultracain, Lidocain, Mepivacain, Bupivacain, Prilocain, Articain, Procain und/oder Tetracain auf. Dabei beträgt die Menge des Ultracains, Lidocains und/oder Mepivacains vorzugsweise etwa 0,1 bis 0,3 %, insbes. etwa 0,2 %, bezogen auf den Trockengehalt der Kollagenmatrix. Bupivacain wird zweckmäßigerweise in einer Menge von 0,25 bis 0,75 %, insbes. etwa 0,5 %, bezogen auf den Trockengehalt der Kollagenmatrix, zugesetzt. Gleiches gilt für Prilocain. Articain, Procain und Tetracain gelten mittlerweile als veraltet und werden nur noch bei allergischen Reaktionen gegen den Amintyp verwendet. Dabei beträgt die Menge zweckmäßigerweise 0,5 bis 1 %, bezogen auf den Trockengehalt der Kollagenmatrix.

Üblicherweise werden Lokalanästhetika intramuskulär verabreicht oder oberflächlich in Form eines Gels auf das Zahnfleisch bzw. die Mundschleimhaut aufgetragen. Dabei ist es nachteilhaft, daß sich das Gel insbes. bei der Behandlung von Aphthen, die meist an der Unterlippe auftreten, schnell und stark im Mundraum verteilt. Hierdurch kommt es zu einer unangenehmen, weiträumigen Betäubung im Mundbereich. Dieses Problem kann durch den Einsatz einer biodegradierbaren Kollagenmatrix als Trägermaterial für das Lokalanästhetikum gelöst werden. Dabei wird das Lokalanästhetikum kontinuierlich an die Mundschleimhaut abgegeben. Es kommt somit nur zu einer lokalen Betäubung der Stelle, die auch wirklich betäubt werden soll. Das als Träger benutzte bioresorbierbare Material, wie etwa Kollagen, löst sich nicht so schnell auf wie ein ansonsten eingesetztes Gel, so daß mit vergleichsweise geringer Wirkstoffdosierung über einen längeren Zeitraum eine gute Betäubung des zu behandelnden Areals erreicht werden kann.

Ferner ist als weiterer Vorteil der Verwendung eines Lokalanästhetikums hervorzuheben, daß der Einsatz einer Spritze beim Zahnarzt den meisten Kindern, aber auch Erwachsenen, Angst macht. Ein Lokalanästhetikum, das zuvor an der Einstichstelle angewendet wird, bewirkt die Unterdrückung des Penetrationsschmerzes. Hierdurch verbinden Kinder diesen Moment nicht mehr mit einem schlechten Erlebnis und es kommt nicht zu einer Traumatisierung. Lokalanästhetika sind zwar verfügbar, sollen aber häufig nicht bei Kindern unter 12 Jahren verwendet werden. Außerdem schmecken sie nicht besonders gut. Eine Kollagenauflage gibt nur einen Bruchteil der Menge des Lokalanästhetikums ab, insbes. wenn es unter kontrollierten Bedingungen von einem Zahnarzt eingesetzt wird. Hierdurch kann eine solche Kollagenmatrix auch bei Kindern zum Einsatz kommen. Außerdem ist davon auszugehen, daß ein kleines Stück Kollagenmatrix mit einem Lokalanästhetikum, das beispielsweise süß schmeckt, von Kindern besser akzeptiert wird als eine Salbe oder ein Gel.

Die Konzentration des Antiseptikums bzw. Lokalanästhetikums liegt zweckmäßigerweise im Rahmen der physiologischen Wirksamkeit im Bereich von 0,2 bis 2 %, bezogen auf das Trockengewicht des Trägermaterials. Gemäß einem weiteren Gesichtspunkt der Erfindung kann das erfindungsgemäße Produkt einen eine antimikrobielle Verbindung aufweisenden Begleitstoff enthalten. Die antimikrobielle Verbindung kann aus dem Bereich der Biguanide stammen. Dabei kann es sich im besonderen um Polyhexamethylenbiguanid Hydrochlorid, Chlorhexidin Dihydrochlorid, Chlorhexidin Diacetat, Chlorhexidin D-Glukonat, Octenidin, Taurolidin, Cetylpyridiniumhydrochlorid und/oder Mischungen daraus handeln.

Häufig sind Wunden im Mundbereich infiziert bzw. es ist eine mikrobiologische Besiedlung der Wunde ursächlich für die Entstehung der Wunde. Als Beispiel dafür ist eine Traumatisierung bzw. eine Druckstelle zu nennen, die sich jederzeit in eine infizierte Wunde verwandeln kann. Weitaus häufiger ist im Dentalbereich jedoch Soor oder das Auftreten von Aphthen. Hierbei handelt es sich um eine Wundinfektion.

Wie eingangs bereits erläutert, besteht ein Problem der bekannterweise eingesetzten Präparate zur Behandlung dieser Probleme in der örtlichen Gabe von antiseptischen Substanzen. Ein Gel verteilt sich schnell im Mundraum und führt zu einer negativen Beeinflussung des Mikroklimas im Mundraum. Durch die Verteilung wird schnell eine zu geringe Wirkstoffkonzentration an der zu behandelnden Stelle erreicht. Dies führt zu einer unzureichenden bzw. keiner antimikrobiellen Wirksamkeit des Antiseptikums. Gleiches gilt für Gurgellösungen oder Gele, die bei einer Parodontosebehandlung zum Einsatz kommen. So wird bei einer Parodontosebehandlung ein antimikrobiell wirkendes Gel in eine passende Gebißform gegeben. Diese muß zuvor jedoch hergestellt worden sein. Dies generiert für die Kostenträger besonders hohe Kosten. Der Effekt, der hierdurch erreicht wird, ist eine antimikrobielle Wirkung über einen längeren Zeitraum, da sich das Gel durch die Gebißform nicht verteilen kann.

Eine Lösung für die genannten Verteilungs- bzw. Kostenprobleme stellt die erfindungsgemäß einsetzbare biodegradierbare Kollagenmatrix dar. Mit einer solchen Matrix wird an der infizierten Wunde kontinuierlich Wirkstoff freigesetzt. Aufgrund der festen Struktur des Trägermaterials bzw. der Kollagenmatrix kann es zu keiner Verteilung der Auflage kommen. Daher kann eine Wundinfektion für die Mundflora schonender und, durch eine längere Verweilzeit des Produktes, effektiver angegangen werden.

Sehr wirksame antimikrobielle Substanzen mit einer Breitbandwirkung gegenüber einer Vielzahl von Mikroorganismen stellen die Biguanide dar. Diese wirken auch gut gegenüber multiresistenten Keimen. Biguanide weisen nicht nur eine gute antibakterielle Wirkung auf, sondern auch eine sehr geringe Zytotoxizität. Dabei wird Polyhexanit als Begleitstoff zweckmäßigerweise in einer Menge von 0,5 bis 1 %, bezogen auf den Trockengehalt der Kollagenmatrix, eingesetzt. Chlorhexidin wird zweckmäßigerweise in einer Menge von 0,5 bis 1,5 %, insbes. etwa 1 %, bezogen auf den Trockengehalt der Kollagenmatrix, eingesetzt. Gleiches gilt für andere Chlorhexidine. Octenidin, Taurolidin und Cetylpyridiniumhydrochlorid werden ebenfalls zweckmäßigerweise in einer Menge von 0,5 bis 1,5 %, insbes. in einer Menge von etwa 1 %, bezogen auf den Trockengehalt der Kollagenmatrix, eingesetzt.

Die Konzentration der als Begleitstoff eingesetzten antimikrobiellen Verbindungen beträgt erfindungsgemäß im Rahmen der antimikrobiellen Wirksamkeit insgesamt zweckmäßigerweise 0,5 bis 2 %, bezogen auf das Trockengewicht des Trägermaterials.

Die antimikrobiellen Verbindungen können auch silberhaltige Verbindungen aufweisen. Dabei kann es sich um ein lösliches Silbersalz, ein schwerlösliches Silbersalz, Silberkomplexe, organische Silberverbindungen und/oder elementares Silber, vorzugsweise um Silberchlorid, handeln. Diese silberhaltigen Verbindungen können als Silbernitrat, Silberacetat, Silberkarbonat, Silberhalogenid, Silbersulfatiacin, Silberdiamin-Komplexe, Silberdithionat-Komplexe, Silberthiocyanat, Silberpulver und/oder Mischungen daraus vorliegen.

Bei der Behandlung infizierter Wunden zeigen Silberionen eine gute antimikrobielle Wirkung. Schon in geringer Konzentration (1 x 10⁻⁶ bis 1 x 10⁻⁹ Mol/l) sind Silberionen sehr wirksam zur Bekämpfung von Bakterien, Viren und Parasiten. Dabei werden häufig Silberprodukte mit einer geringen Löslichkeit eingesetzt, die dadurch bedingt nur geringe Mengen Silberionen freisetzen. Dazu gehört Silberchlorid, Silberbromid, Silberjodid, Silberoxid, Silberkarbonat, Silberthiocyanat und auch Silberpulver. Diese Substanzen geben nach ihrer Applikation kontinuierlich geringe Mengen Silber aus der Auflage frei. Die Silberionen werden bei ihrer antimikrobiellen Wirkung zu elementarem Silber reduziert. Hierdurch sinkt schnell die Konzentration der Silberionen in der Wundflüssigkeit. Da nur Silberionen eine antimikrobielle Wirkung aufweisen, ist es wesentlich, daß über einen längeren Zeitraum Silberionen in ausreichender Konzentration zur Verfügung stehen. Durch den Einsatz von schwer löslichen Silbersalzen werden gemäß dem chemischen Gleichgewicht kontinuierlich Silberionen nachgeliefert, solange noch Silbersalze in der Wundauflage vorhanden sind.

Eine infizierte Wunde benötigt am Anfang der Behandlung eine größere Menge Silber und im späteren Verlauf, nachdem die Keimzahl reduziert wurde, eine kontinuierliche Silberionenfreigabe. Dies kann erfindungsgemäß durch eine Mischung von Silber-Komplexen und schwerlöslichen Silberverbindungen in der polymeren Matrix erreicht werden. Hierbei werden zunächst die Silberkomplexe aus der Kollagenmatrix herausgelöst. Dadurch kommt es zu einer Stoßfreisetzung von Silber in der Wundflüssigkeit. Ein Zusatz an schwerlöslichen Silbersalzen sorgt für eine kontinuierliche Freigabe von Silber-lonen über einen langen Zeitraum. Bei einem Ausführungsbeispiel werden in 1000 ml einer 1 % Kollagenmatrixlösung (Kollagen und weitere Hilfs- bzw. Zusatzstoffe) 5 ml einer Silberacetatlösung gegeben. Die Lösung enthält 15,46 mg Silberacetat. Nachdem die Lösung homogenisiert wurde, wurden 5 ml einer Natriumchloridlösung hinzugefügt. Die Lösung enthielt 5,4 mg Natriumchlorid. Alternativ kann auch erst die Silberacetatlösung mit der Natriumchloridlösung gemischt werden, die anschließend der Kollagendispersion zugegeben wird. Eine Zugabe von 29,85 mg Silberdithionat (Ag[S₂O₃]₂) ist im Anschluß möglich. Silberkonzentration: 0,1 % AgCl und 0,1 % Silber-Komplex.

Die Konzentration der silberhaltigen Verbindungen liegt im Rahmen der antimikrobiellen Wirksamkeit erfindungsgemäß zweckmäßigerweise im Bereich von 0,1 bis 2 %, bezogen auf das Trockengewicht des Trägermaterials.

Wie eingangs bereits erläutert, weist das bioresorbierbare Material zweckmäßigerweise einen feinporigen Schwamm auf, mit dem eine besonders gute Polsterwirkung erhalten werden kann. Durch die Porenstruktur nimmt die vorzugsweise als bioresorbierbares Material eingesetzte Kollagenmatrix viel Flüssigkeit auf. Ferner wird durch die schaumartige Struktur eine gute Kompressibilität erreicht. Der Schaum füllt daher besonders gut unebene oder zerklüftete Stellen aus. Ein trockener Kollagenschwamm haftet spontan am Zahnfleisch bzw. den Zähnen. Es hat sich empirisch gezeigt, daß ein filmartiges Material bei der Anwendung im Oralbereich deutlich schlechter haftet.

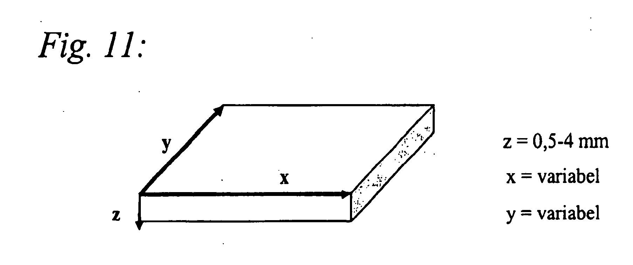

Zum Erhalt einer zufriedenstellenden Polsterwirkung bei gleichzeitiger Vermeidung einer unangenehmen Füllwirkung hat es sich als zweckmäßig erwiesen, wenn das bioresorbierbare Material einen Schaum bzw. Schwamm aufweist, der in Z-Richtung (vgl. Fig. 11) eine Dicke von 0,5 bis 4 mm aufweist.

Zweckmäßigerweise wird der Schaum in Sonderformen für den Dentalbereich geformt.

Als besonders vorteilhaft hat es sich erwiesen, wenn das erfindungsgemäße Produkt durch Strahlung (25 kGy) oder durch Begasen mit Ethylenoxid sterilisierbar ist.

Zusätzlich oder alternativ kann das bioresorbierbare Material im Rahmen der Erfindung, vorzugsweise bedingt durch einen Lyophilisationsprozeß, auch noch eine gute Verformbarkeit in X- und Y-Richtung (vgl. Fig. 11) aufweisen.

Im Rahmen der Verträglichkeit erfindungsgemäßer Produkte ist es angestrebt, daß das bioresorbierbare Material im Dentalbereich abbaubar ist und bei einem versehentlichen Verschlucken vollständig resorbiert wird. Zur Erhöhung der Haltbarkeit kann die Biodegradierbarkeit des bioresorbierbaren Materials durch zumindest teilweise Vernetzung verringert werden. Als zweckmäßig hat es sich erwiesen, wenn das bioresorbierbare Material mit bi- bzw. multifunktionellen Aldehyden, Carbonsäuren, Carbonsäurechloriden, Carbonsäurealdehyden und/oder Epoxiden vernetzbar ist. Zusätzlich oder alternativ kann die Biodegradierbarkeit des bioresorbierbaren Materials zweckmäßigerweise mit Hilfe spaltbarer Brükken sowie durch γ-Bestrahlung oder durch β-Bestrahlung verändert werden.

Wie der vorstehenden Erläuterung erfindungsgemäßer Produkte zu entnehmen ist, wird das bioresorbierbare Material im Rahmen der Erfindung zur Wundheilung und/oder Desinfektion (z.B. Behandlung von Aphthen, Druckstellen, Mykosen, Soor und anderen Infektionen) eingesetzt. Dazu kann das bioresorbierbare Material als Polster zwischen Zahnersatz bzw. Zahnspangen und der Mundschleimhaut, einer Wunde oder dem Zahnfleisch eingesetzt werden. Zusätzlich oder alternativ kann das bioresorbierbare Material auch als Haftmittel für Voll- oder Teilprothesen eingesetzt werden, wobei es ggf. durch Chitosan-Zusatz zur Hämostase bei Blutungen im Dentalbereich (auch bei Personen die unter dem Einfluß von Blutgerinnungshemmern, wie z.B. Marcumar, stehen) eingesetzt wird.

Vorstehend wurde bereits darauf hingewiesen, daß das erfindungsgemäß eingesetzte bioresorbierbare Material eine gute Haftung an der Mundschleimhaut aufweist. Wenn eine kollagenhaltige Auflage im Mundraum plaziert wird, klebt diese sehr gut an Zähnen und Zahnfleisch. Zur quantitativen Bestimmung der Haftung wurden folgende Untersuchungen durchgeführt:

Ein Objektträger wurde mit einer 2,5 % warmen Agar-Lösung beschichtet. Die Agar-Lösung wurde komplett eingetrocknet. Die zu untersuchenden Proben wurden mit Speichel befeuchtet und auf einen beschichteten Objektträger gegeben. Ein zweiter beschichteter Objektträger wurde nach dem Bespeicheln der Probe an die Probe herangefahren (Abstand: 9,6 mm). Es wurde ein Kontakt von mind. einer Minute aufrechterhalten, bevor eine Zugmessung durchgeführt wurde. Die Probe weist eine Fläche von 15 mm x 20 mm auf. Die zur Ablösung der Probe von dem Objektträger erforderliche Zugkraft (Haftung) beträgt bei mit 10% Na-und 10% Ca-Alginat als zuzätlichem bioresorbierbaren Material versetztem Kollagen 4,5 N. Bei mit 60% Honig, 5% Propolis und 10% Na-Alginat versetztem Kollagen beträgt die Haftung 3,7 N.

Ferner wurden Untersuchungen zur Bestimmung der Kompressibilität unterschiedlicher Kollagenauflagen durchgeführt. Dazu wurden die Kollagenauflagen (bioresorbierbares Material) für 10 Sekunden mit einem Druck von 1 bar belastet. Es wurde die Dicke vor und nach der Belastung bestimmt. Außerdem wurde die Probe zwischen Zeigefinger und Daumen für einige Sekunden fest zusammengedrückt, um die maximale Kompressibilität einer trockenen Auflage zu bestimmen. Die Ergebnisse sind in der folgenden Tabelle dargestellt:

| Bezeichnung | Dicke unbelastet | Dicke 10 Sek. 1 bar | Dicke 10 Sek. > 5 bar | Bemerkung |
|---|---|---|---|---|
| Subrasorb C (Referenz) | 7,1 mm | 1,7 mm | 0,35 mm | reversibel |
| Kollagen + 10 % Na-Alginat | 6,0 | 0,8 | 0,27 | irreversibel |
| Kollagen + 10 % Na-Alginat + 60 % Honig + 5 % Propolis | 6,1 | 0,33 | 0,20 | irreversibel |
| Kollagen + 10 % Na- + 10 % Ca-Alginat | 7,0 | 1,2 | 0,45 | reversibel |

Bei dem in der Tabelle angegebenen Referenzprodukt "Subrasorb C" handelt es sich um ein Produkt, das gemäß DE 38 32 162 C2 hergestellt wurde.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, näher erläutert. In der Zeichnung zeigt:
- **Fig. 1A**: ein erfindungsgemäßes Produkt für die Anwendung im Zusammenhang mit Vollprothesen im Unterkiefer,
- **Fig. 1B**: ein erfindungsgemäßes Produkt für die Anwendung im Zusammenhang mit Vollprothesen im Oberkiefer,
- **Fig. 2**: ein erfindungsgemäßes Produkt für die Versorgung von Zahnfleisch,
- **Fig. 3A und 3B**: einen z.B. zum Fixieren an einem Zahn einsetzbaren Kollagenschwamm mit einem Band,
- **Fig. 4**: einen eingeschnittenen Kollagenschwamm zum Abreißen,
- **Fig. 5**: einen zur Herstellung erfindungsgemäßer Produkte einsetzbaren Kollagenstreifen,
- **Fig. 6**: ein erfindungsgemäßes Produkt in Form eines U-förmigen Kollagenschwämmchens zur Versorgung eines Zahns,
- **Fig. 7**: einen Spender zur Bereitstellung erfindungsgemäßer Produkte,
- **Fig. 8**: ein erfindungsgemäßes Produkt zur Versorgung des Zahnfleisches über einigen Zähnen,
- **Fig. 9**: ein erfindungsgemäßes Produkt zur Anwendung im Zusammenhang mit Vollprothesen im Oberkiefer gemäß einer weiteren Ausführungsform der Erfindung,
- **Fig. 10**: ein variabel einsetzbares erfindungsgemäßes Produkt in ovaler Form und
- **Fig. 11**: eine 3-D-Darstellung des erfindungsgemäß einsetzbaren biodegradierbaren Materials.

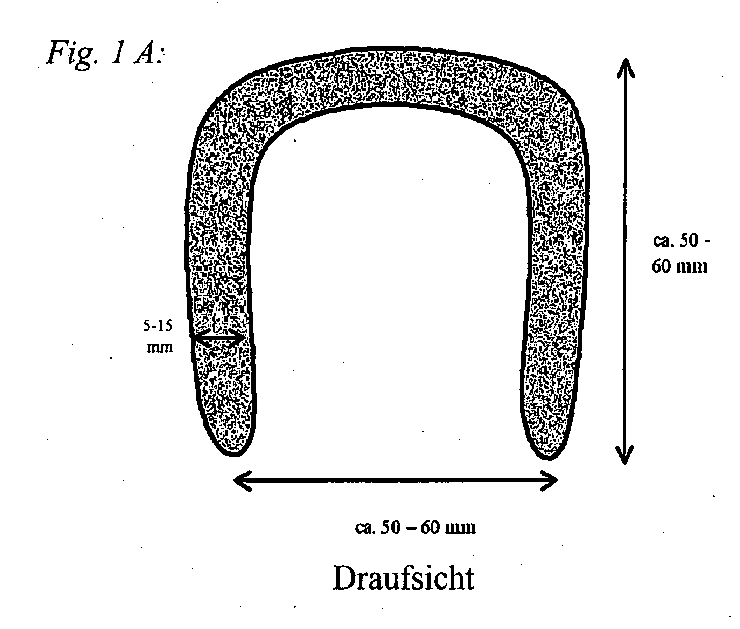

Das in Fig. 1A dargestellte Produkt zum Einsatz im Zusammenhang mit Vollprothesen im Unterkiefer ist im wesentlichen U-förmig ausgeführt. Es weist eine Breite zwischen den Schenkeln von etwa 50 bis 60 mm auf und eine Höhe von ebenfalls etwa 50 bis 60 mm. Die Materialstärke der Schenkel beträgt etwa 5 bis 15 mm.

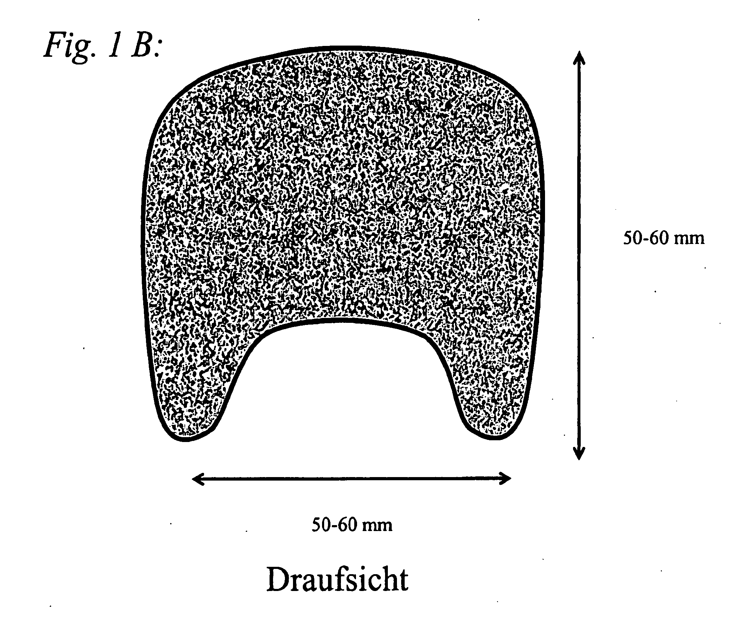

Das in Fig. 1B dargestellte Produkt entspricht hinsichtlich der äußeren Form im wesentlichen dem in Fig. 1A dargestellten Produkt. Es ist jedoch zwischen den Schenkeln ausgefüllt, so daß es den Raum zwischen einer Vollprothese im Oberkiefer und dem Gaumen nahezu vollständig ausfüllen kann.

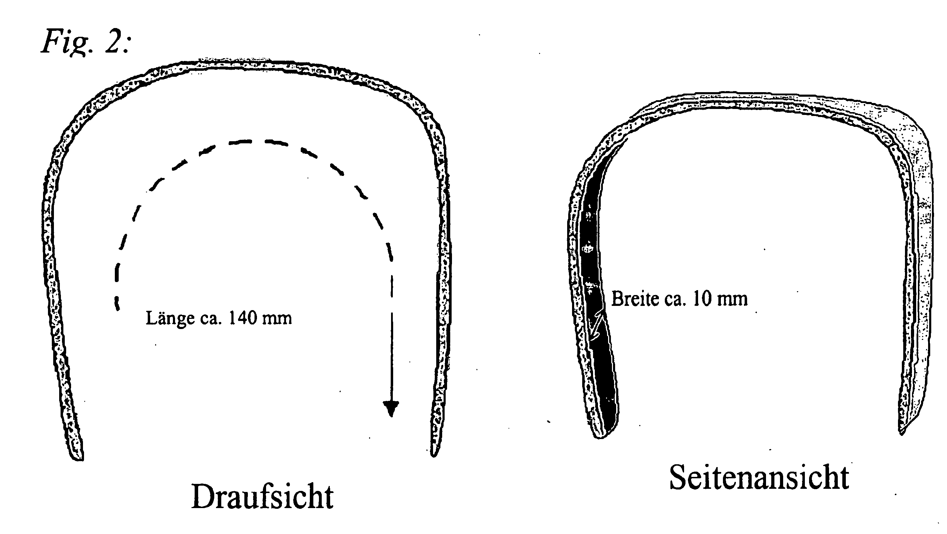

Das in Fig. 2 dargestellte erfindungsgemäße Produkt zur Versorgung von Zahnfleisch ist ebenfalls im wesentlichen U-förmig ausgeführt. Es weist eine Länge von etwa 140 mm auf, wobei die Materialstärke gemäß der Seitenansicht etwa 10 mm beträgt.

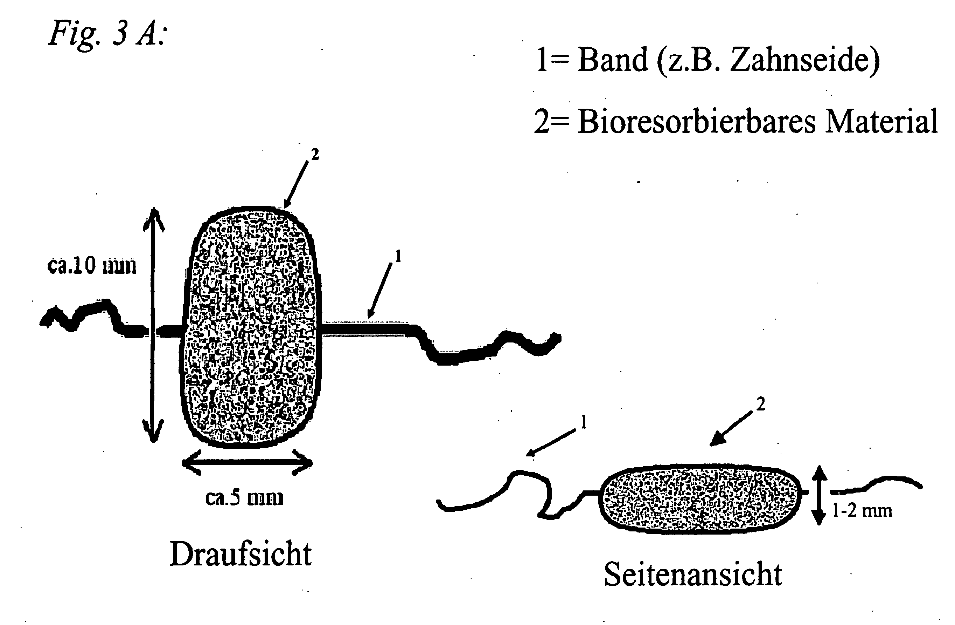

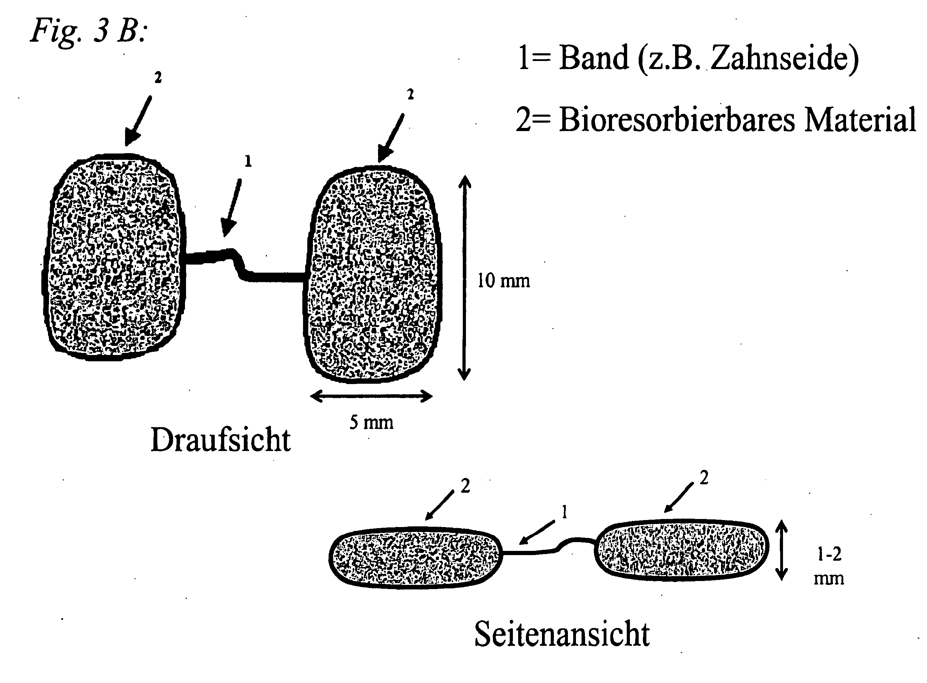

Das in Fig. 3 dargestellte Produkt zur Versorgung einzelner Zähne weist einen Kollagenschwamm mit einer Höhe von etwa 10 mm und einer Breite von etwa 5 mm auf, wobei die Dicke des Kollagenschwamms etwa 1 bis 2 mm beträgt. Wie besonders deutlich in Fig. 3A zu erkennen ist, sind an dem Kollagenschwamm Fixierungsbänder befestigt, die beispielsweise aus Zahnseide gebildet sein können. Gemäß Fig. 3B können auch 2 oder mehr Kollagenschwämme der in Fig. 3A dargestellten Art zur Versorgung unterschiedlicher Bereiche eines Zahns und/oder zur Versorgung nebeneinanderliegender Zähne mit Hilfe von beispielsweise aus Zahnseide gebildeten Fixierungsbändern miteinander verbunden sein.

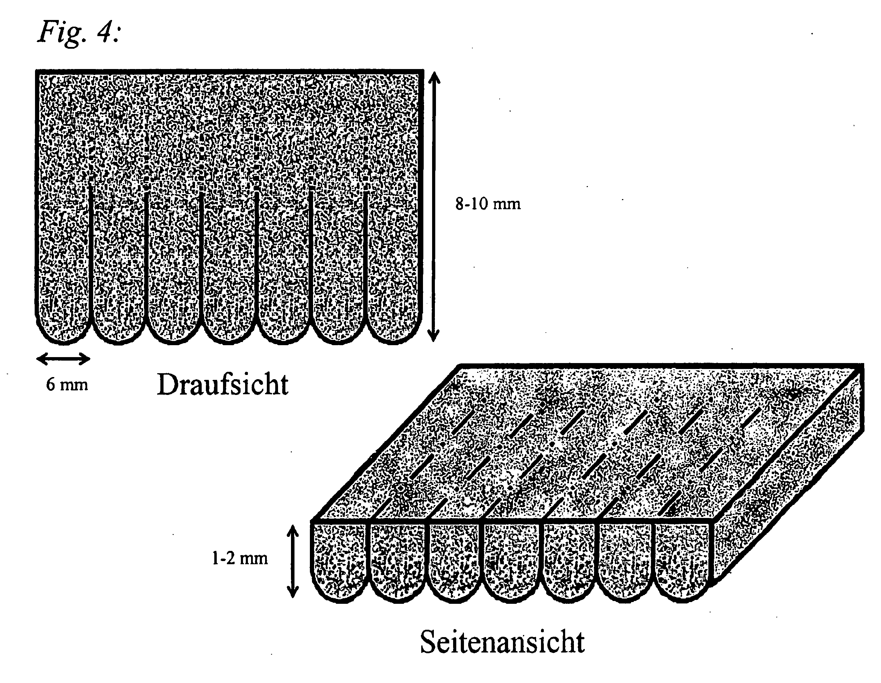

Bei dem in Fig. 4 dargestellten eingeschnittenen Kollagenschwamm zur Herstellung erfindungsgemäßer Produkte handelt es sich um einen Kollagenstreifen mit beliebiger Länge, einer Breite von etwa 8 bis 10 mm und einer Dicke von etwa 1 bis 2 mm. Wie besonders deutlich in der Seitenansicht zu erkennen ist, weist dieser Kollagenstreifen senkrecht zur Längsrichtung geführte Einschnitte auf, die einen Abstand von etwa 6 mm voneinander aufweisen. Der so vorbereitete Streifen kann beispielsweise zur Herstellung von in Fig. 3 dargestellten Produkten eingesetzt werden.

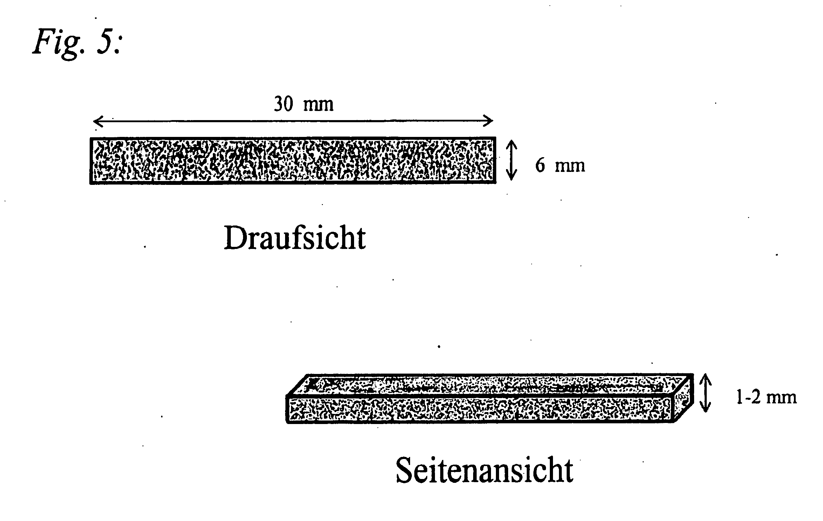

Der in Fig. 5 dargestellte Kollagenstreifen kann beispielsweise zur Herstellung von erfindungsgemäßen Produkten gemäß Fig. 2 eingesetzt werden.

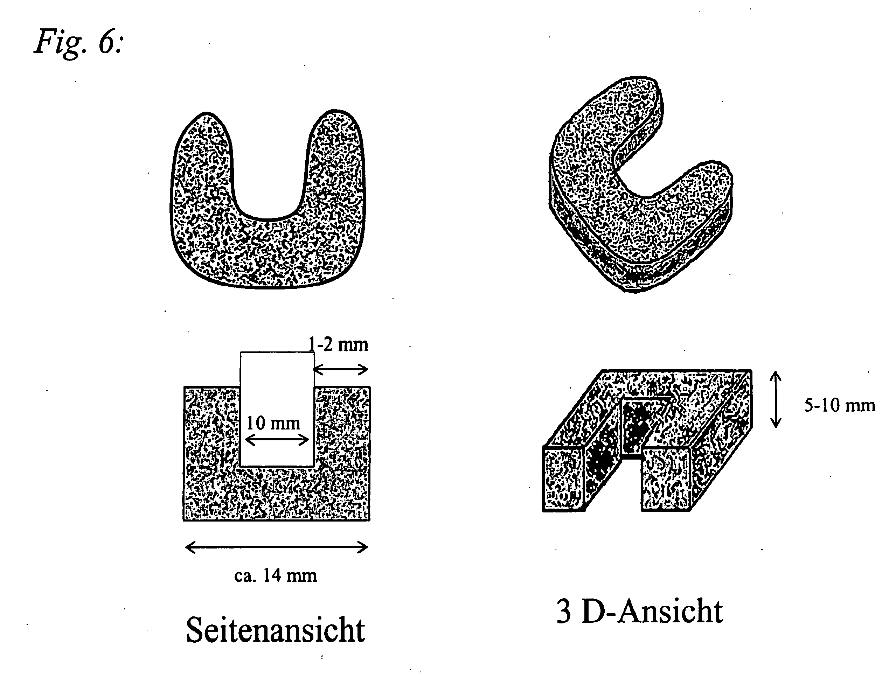

Zusätzlich oder alternativ kann der in Fig. 5 dargestellte Kollagenstreifen auch zur Herstellung von in Fig. 6 dargestellten Produkten eingesetzt werden.

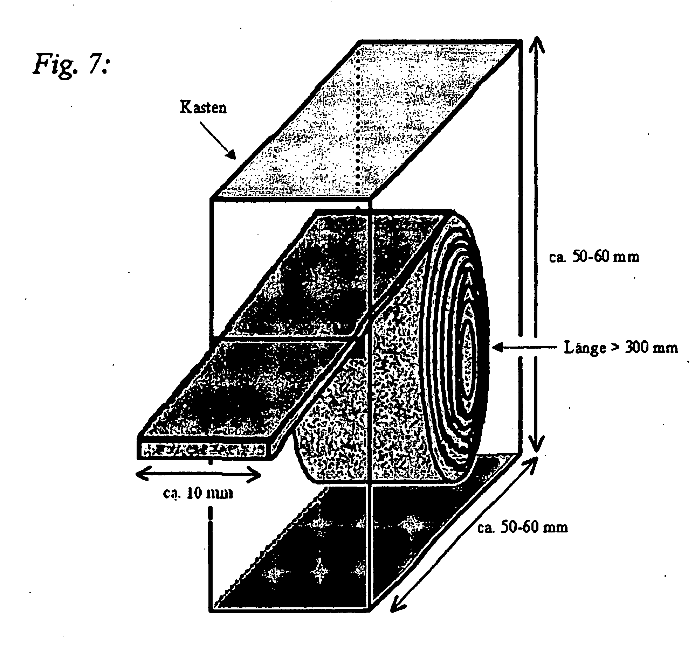

Der in Fig. 5 dargestellte Kollagenstreifen kann beispielsweise spiralförmig aufgewickelt und in einem Spender gemäß Fig. 7 untergebracht werden.

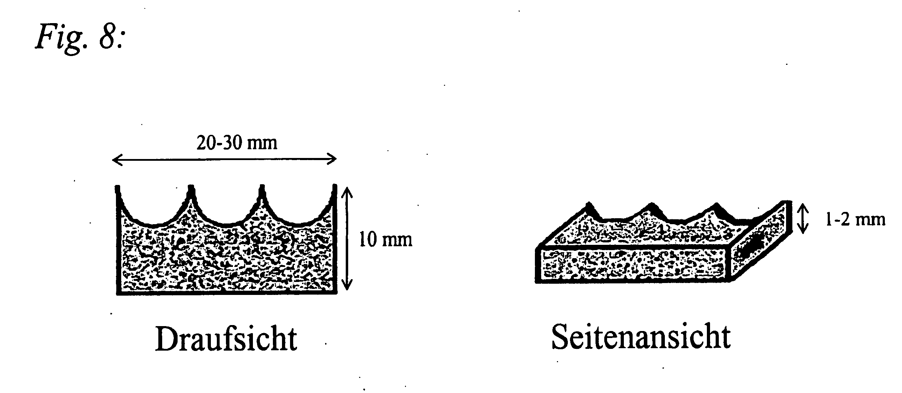

Bei dem in Fig. 8 dargestellten Produkt zur Versorgung des Zahnfleisches über einigen Zähnen ist eine Längskante des Streifens wellenförmig ausgeführt, um so eine Anpassung an die Zahneinschnitte im Zahnfleisch herbeizuführen.

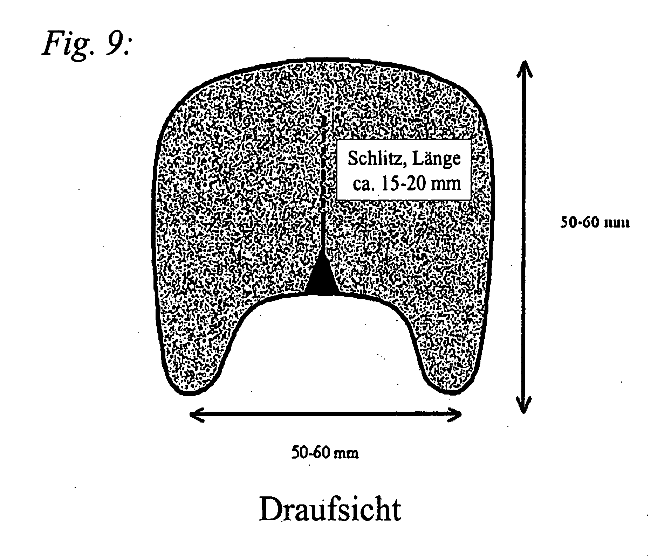

Bei dem in Fig. 9 dargestellten Produkt handelt es sich um eine Sonderform des in allgemeiner Form in Fig. 1B dargestellten Produktes, bei dem durch einen Einschnitt eine bessere Anpassung an die Prothese im Oberkiefer erreichbar ist.

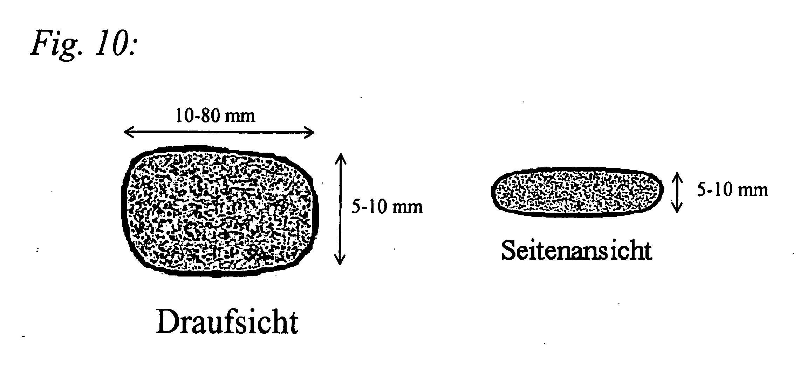

Das in Fig. 10 dargestellte Produkt ist im Mundraum universell einsetzbar.

Das in Fig. 11 dargestellte Produkt dient zur Erläuterung der vorstehend beschriebenen Kompressibilität erfindungsgemäßer Produkte.

Bei den vorstehend herausgestellten und in der Zeichnung angegebenen Bemessungsangaben handelt es sich um im Rahmen der Erfindung bevorzugte Bereiche. Es kann allerdings auch von diesen Bereichen abgewichen werden. Das gilt insbes. für die Anwendung erfindungsgemäßer Produkte bei Kindern. Bei dieser Anwendung können die in der Zeichnung dargestellten Formen erhalten bleiben, es werden jedoch die Abmessungen entsprechend verkleinert.

## Patentansprüche

1. Produkt zur Versorgung von Entzündungen, Druckstellen und/oder Aphthen im Oralbereich aus einem überwiegend bioresorbierbaren und Kollagen enthaltenden Material, **dadurch gekennzeichnet, daß** das bioresorbierbare Material ein an der Schleimhaut haftendes, flexibles und kompressibles Polsterelement aufweist und durch eine kurze Druckeinwirkung von 1 bar in Z-Richtung (vgl. Fig. 11) auf weniger als 50 % der ursprünglichen Dicke komprimierbar ist und zusätzlich zu Kollagen ein Alginat als weiteres bioresorbierbares Material aufweist.

2. Produkt nach Anspruch 1, **dadurch gekennzeichnet, daß** das bioresorbierbare Material ein mit mindestens einem Begleitstoff versetztes Kollagen aufweist.

3. Produkt nach Anspruch 2, **dadurch gekennzeichnet, daß** das Kollagen bovines, equines, porkines und/oder marines Kollagen aufweist.

4. Produkt nach einem der Ansprüche 2 bis 3, **dadurch gekennzeichnet, daß** das bioresorbierbare Material mindestens einen die Erhöhung der Haftung an der Schleimhaut, die Verbesserung der Flexibilität bzw. Anschmiegsamkeit und/oder die Verbesserung des Geschmacks bewirkenden Begleitstoff aufweist.

5. Produkt nach Anspruch 4, **dadurch gekennzeichnet, daß** der Begleitstoff Honig, Propolis, Aloe Vera, Panthenol, Polyethylenglykol 200-2000, Polyvinylpyrrolidon, Glycerin, Kamilleextrakt, Salbeiöl, Pfefferminzöl, Orangenextrakt, Zitronenextrakt, Cyclamat, aromatische Süßstoffe, Vitamine und/oder Provitamine aufweist.

6. Produkt nach einem der Ansprüche 4 oder 5, **dadurch gekennzeichnet, daß** der Begleitstoff in einer Konzentration von 0,5 bis 50 %, bezogen auf das Trockengewicht des Trägermaterials, vorliegt.

7. Produkt nach einem der Ansprüche 2 bis 6, **dadurch gekennzeichnet, daß** der Begleitstoff ein Anästhetikum und/oder Antiseptikum aufweist.

8. Produkt nach Anspruch 7, **dadurch gekennzeichnet, daß** das Antiseptikum bzw. Lokalanästhetikum aus der Gruppe der Ester bzw. Säureamide der Paraaminobenzoesäure ausgewählt ist.

9. Produkt nach Anspruch 7 oder 8, **dadurch gekennzeichnet, daß** dem Antiseptikum bzw. Lokalanästhetikum in einer Verdünnung von 1 : 200.000 Adrenalin zur Wirkstoffverbesserung zugesetzt ist.

10. Produkt nach einem der Ansprüche 7 bis 9, **dadurch gekennzeichnet, daß** das Anästhetikum Ultracain, Lidocain, Mepivacain, Bupivacain, Prilocain, Articain, Procain und/oder Tetracain aufweist.

11. Produkt nach einem der Ansprüche 7 bis 10, **dadurch gekennzeichnet, daß** die Konzentration des Antiseptikums bzw. Lokalanästhetikums im Rahmen der physiologischen Wirksamkeit im Bereich von 0,1 bis 2 %, bezogen auf das Trockengewicht des Trägermaterials, liegt.

12. Produkt nach einem der Ansprüche 2 bis 11, **gekennzeichnet durch** mindestens einen eine antimikrobielle Verbindung aufweisenden Begleitstoff.

13. Produkt nach Anspruch 12, **dadurch gekennzeichnet, daß** die antimikrobielle Verbindung aus dem Bereich der Biguanide stammt.

14. Produkt nach Anspruch 13, **dadurch gekennzeichnet, daß** der Begleitstoff Polyhexamethylenbiguanid Hydrochlorid, Chlorhexidin Dihydrochlorid, Chlorhexidin Diacetat, Chlorhexidin D-Glukonat, Octenidin, Taurolidin, Cetylpyridiniumhydrochlorid und/oder Mischungen daraus aufweist.

15. Produkt nach einem der Ansprüche 12 bis 14, **dadurch gekennzeichnet, daß** die Konzentration der Begleitstoffe im Rahmen der antimikrobiellen Wirksamkeit im Bereich von 0,5 bis 2 %, bezogen auf das Trockengewicht des Trägermaterials, liegt.

16. Produkt nach einem der Ansprüche 12 bis 15, **dadurch gekennzeichnet, daß** die antimikrobielle Verbindung eine silberhaltige Verbindung aufweist.

17. Produkt nach Anspruch 16, **dadurch gekennzeichnet, daß** die silberhaltige Verbindung mindestens ein lösliches Silbersalz, schwerlösliches Silbersalz, Silberkomplexe, organische Silberverbindungen und/oder elementares Silber, vorzugsweise Silberchlorid, umfaßt.

18. Produkt nach Anspruch 16 oder 17, **dadurch gekennzeichnet, daß** die silberhaltigen Verbindungen Silbernitrat, Silberacetat, Silberkarbonat, Silberhalogenide, Silbersulfatiacin, Silberdiamin-Komplexe, Silberdithionat-Komplexe, Silberthiocyanat, Silberpulver und/oder Mischungen daraus aufweisen.

19. Produkt nach einem der Ansprüche 15 bis 18, **dadurch gekennzeichnet, daß** die Konzentration der Begleitstoffe im Rahmen der antimikrobiellen Wirksamkeit im Bereich von 0,1 bis 2 %, bezogen auf das Trockengewicht des Trägermaterials, liegt.

20. Produkt nach einem der vorhergehenden Ansprüche, **dadurch gekennzeichnet, daß** das bioresorbierbare Material einen feinporigen Schwamm aufweist.

21. Produkt nach einem der vorhergehenden Ansprüche, **dadurch gekennzeichnet, daß** das bioresorbierbare Material einen Schaum bzw. Schwamm aufweist, der in Z-Richtung (vgl. Fig. 11) eine Dicke von 0,5 bis 4 mm aufweist.

22. Produkt nach einem der vorhergehenden Ansprüche, **dadurch gekennzeichnet, daß** es durch Strahlung von 25 kGy oder durch Begasen mit Ethylenoxid sterilisierbar ist.

23. Produkt nach einem der vorhergehenden Ansprüche, **dadurch gekennzeichnet, daß** das bioresorbierbare Material im Dentalbereich abbaubar ist und bei einem versehentlichen Verschlucken vollständig resorbiert wird.

24. Produkt nach einem der vorhergehenden Ansprüche, **dadurch gekennzeichnet, daß** das bioresorbierbare Material zur Verringerung der Biodegradierbarkeit zumindest teilweise vernetzt ist.

25. Produkt nach einem der vorhergehenden Ansprüche, **dadurch gekennzeichnet, daß** das bioresorbierbare Material mit bi- bzw. multifunktionellen Aldehyden, Carbonsäuren, Carbonsäurechloriden, Carbonsäurealdehyden und/oder Epoxiden vernetzbar ist.

26. Produkt nach einem der vorhergehenden Ansprüche, **dadurch gekennzeichnet, daß** das bioresorbierbare Material mit spaltbaren Brücken oder durch γ-Bestrahlung oder durch β-Bestrahlung hinsichtlich der Biodegradierbarkeit veränderbar ist.

27. Produkt nach Anspruch 26, **dadurch gekennzeichnet, daß** als spaltbare Vernetzungsbrücken Hydroxyethyl methacrylate einsetzbar sind.

28. Produkt nach einem der vorhergehenden Ansprüche, **dadurch gekennzeichnet, daß** das bioresorbierbare Material zur Wundheilung und/oder zur Desinfektion einsetzbar ist.

29. Produkt nach einem der vorhergehenden Ansprüche, **dadurch gekennzeichnet, daß** das bioresorbierbare Material als Polster zwischen Zahnersatz bzw. Zahnspangen und der Mundschleimhaut einer Wunde oder dem Zahnfleisch einsetzbar ist.

30. Produkt nach Anspruch 1, **dadurch gekennzeichnet, daß** das bioresorbierbare Material als Haftmittel für Voll- oder Teilprothesen einsetzbar ist.

31. Produkt nach einem der vorhergehenden Ansprüche, **dadurch gekennzeichnet, daß** es durch Chitosanzusatz zur Hämostase bei Blutungen im Dentalbereich, auch bei Personen, die unter dem Einfluß von Btutgerinnungshemmern, wie z.B. Marcumar, stehen, einsetzbar ist.

32. Produkt nach einem der vorhergehenden Ansprüche, **gekennzeichnet durch** ein Haftungsvermögen von 1 Newton oder mehr.

## Claims

1. A product for attending to inflammations, pressure points and/or aphthae in the oral region made of a material that is predominantly bioresorbable and containing collagen, **characterised in that** the bioresorbable material has a flexible and compressible cushioning element adhering to the mucous membrane and due to a short pressure effect of 1 bar in the Z direction (see Fig. 11) can be compressed over less than 50 % of the original thickness and has an alginate in addition to collagen as a further bioresorbable material.

2. The product according to Claim 1, **characterised in that** the bioresorbable material has a collagen displaced with at least one associated material.

3. The product according to Claim 2, **characterised in that** the collagen has bovine, equine, porcine and/or marine collagen.

4. The product according to either of Claims 2 to 3, **characterised in that** the bioresorbable material has at least one associated material bringing about an increase in the adhesion onto the mucous membrane, an improvement in flexibilty and clingyness and/or an improvement in taste.

5. The product according to Claim 4, **characterised in that** the associated material has honey, propolis, aloe vera, panthenol, polyethylene glycol 200-2000, polyvinylpyrrolidone, glycerine, camomile extract, salvia oil, peppermint oil, orange extract, lemon extract, cyclamate, aromatic sweeteners, vitamins and/or provitamins.

6. The product according to either of Claims 4 or 5, **characterised in that** the associated material is present in a concentration of 0.5 to 50 % in relation to the dry weight of the carrier material.

7. The product according to any of Claims 2 to 6, **characterised in that** the associated material has an anaesthetic and/or an antiseptic.

8. The product according to Claim 7, **characterised in that** the antiseptic or local anaesthetic is chosen from the group of esters or acid amides of paraaminobenzoic acid.

9. The product according to Claim 7 or 8, **characterised in that** adrenalin is added to the antiseptic or local anaesthetic in a dilution of 1 : 200,000 in order to improve the active substance.

10. The product according to any of Claims 7 to 9, **characterised in that** the anaesthetic has ultracain, lidocain, mepivacain, bupivacain, prilocain, articain, procain and/or tetracain.

11. The product according to any of Claims 7 to 10, **characterised in that** the concentration of the antiseptic or local anaesthetic within the framework of the physiological effectiveness comes within the range of 0.1 to 2 % in relation to the dry weight of the carrier material.

12. The product according to any of Claims 2 to 11, **characterised by** at least one associated material having an antimicrobial compound.

13. The product according to Claim 12, **characterised in that** the antimicrobial compound originates from the region of the biguanides.

14. The product according to Claim 13, **characterised in that** the associated material has polyhexamethylene biguanide hydrochloride, chlorohexidine dihydrochloride, chlorohexidine diacetate, chlorohexidine D-gluconate, octenidine, taurolidine, cetylpyridinium hydrochloride and/or mixtures of the latter.

15. The product according to any of Claims 12 to 14, **characterised in that** the concentration of the associated materials within the framework of antimicrobial effectiveness comes within the range of 0.5 to 2 % in relation to the dry weight of the carrier material.

16. The product according to any of Claims 12 to 15, **characterised in that** the antimicrobial compound has a compound containing silver.

17. The product according to Claim 16, **characterised in that** the compound containing silver comprises at least one soluble silver salt, silver salt of low solubility, silver complexes, organic silver compounds and/or elementary silver, preferably silver chloride.

18. The product according to Claim 16 or 17, **characterised in that** the compounds containing silver have silver nitrate, silver acetate, silver carbonate, silver halogenides, silver sulfatiacin, silver diamine complexes, silver dithionate complexes, silver thiocyanate, silver powder and/or mixutres of the latter.

19. The product according to Claims 15 to 18, **characterised in that** the concentration of the associated materials within the framework of antimicrobial effectiveness comes within the range of 0.1 to 2 % in relation to the dry weight of the carrier material.

20. The product according to any of the preceding claims, **characterised in that** the bioresorbable material has a fine-pored sponge.

21. The product according to any of the preceding claims, **characterised in that** the bioresorbable material has a foam or a sponge which has a thickness of 0.5 to 4 mm in the Z direction (see Fig. 11).

22. The product according to any of the preceding claims, **characterised in that** it can be sterilised by means of radiation of 25 kGy or by blowing with ethylene oxide.

23. The product according to any of the preceding claims, **characterised in that** the bioresorbable material can be broken down in the dental region and is totally resorbed by inadvertently swallowing.

24. The product according to any of the preceding claims, **characterised in that** the bioresorbable material is at least partially crosslinked in order to reduce biodegradability.

25. The product according to any of the preceding claims, **characterised in that** the bioresorbable material can be crosslinked with bi- or multifunctional aldehydes, carboxylic acids, carboxylic acid chlorides, carboxylic acid aldehydes and/or epoxides.

26. The product according to any of the preceding claims, **characterised in that** the bioresorbable material can be changed with regard to biodegradability with divisable bridges or by γ radiation or by β radiation.

27. The product according to Claim 26, **characterised in that** hydroxyethyl methacrylates can be used as divisable crosslinking bridges.

28. The product according to any of the preceding claims, **characterised in that** the bioresorbable material can be used for healing wounds and/or for disinfection.

29. The product according to any of the preceding claims, **characterised in that** the bioresorbable material can be used as cushioning between a dental prosthesis or retainers and the oral mucosa of a wound or the gum.

30. The product according to Claim 1, **characterised in that** the bioresorbable material can be used as an adhesive agent for total or partial prostheses.

31. The product according to any of the preceding claims, **characterised in that** it can be used by adding chitosan for haemostasis with bleeding in the dental region, even with people who are under the influence of blood clotting inhibitors, such as e.g. Marcumar.

32. The product according to any of the preceding claims, **characterised by** an adhesive capability of 1 newton or more.

## Revendications

1. Produit de traitement des inflammations, des contusions et/ou des aphtes dans la cavité buccale composé d'un matériau majoritairement biorésorbable renfermant du collagène, **caractérisé en ce que** le matériau biorésorbable présente un élément tampon souple et compressible adhérant à la muqueuse, qu'il peut être compressé sous l'effet d'une brève pression de 1 bar dans la direction Z (voir Fig. 11) pour atteindre moins de 50 % de son épaisseur originale et qu'il présente, en sus du collagène, un autre matériau biorésorbable sous la forme d'un alginate.

2. Produit selon la revendication 1, **caractérisé en ce que** le matériau biorésorbable présente un collagène additionné d'au moins une matière auxiliaire.

3. Produit selon la revendication 2, **caractérisé en ce que** le collagène présente un collagène d'origine bovine, équine, porcine et/ou marine.

4. Produit selon l'une des revendications 2 à 3, **caractérisé en ce que** le matériau biorésorbable présente au moins une matière auxiliaire permettant d'en accroître l'adhérence à la muqueuse, d'en améliorer la souplesse ou la conformabilité et/ou d'en améliorer le goût.

5. Produit selon la revendication 4, **caractérisé en ce que** la matière auxiliaire présente du miel, de la propolis, de l'aloe vera, du panthénol, du polyéthylène glycol 200-2000, de la polyvinylpyrrolidone, de la glycérine, de l'extrait de camomille, de l'huile de sauge, de l'huile de menthe poivrée, de l'extrait d'orange, de l'extrait de citron, du cyclamate, des édulcorants aromatiques, des vitamines et/ou provitamines.

6. Produit selon l'une des revendications 4 ou 5, **caractérisé en ce que** la matière auxiliaire est présent dans une concentration allant de 0,5 à 50 % par rapport au poids sec du véhicule.

7. Produit selon l'une des revendications 2 à 6, **caractérisé en ce que** la matière auxiliaire présente un anesthésique et/ou un antiseptique.

8. Produit selon la revendication 7, **caractérisé en ce que** l'antiseptique ou l'anesthésique local est choisi dans le groupe des esters ou des amides acides de l'acide paraaminobenzoïque.

9. Produit selon la revendication 7 ou 8, **caractérisé en ce que** l'antiseptique ou l'anesthésique local est additionné d'adrénaline, dans un rapport de dilution de 1 : 200.000, afin d'en améliorer l'efficacité.

10. Produit selon l'une des revendications 7 à 9, **caractérisé en ce que** l'anesthésique présente de l'ultracaïne, de la lidocaïne, de la mépivacaïne, de la bupivacaïne, de la prilocaïne, de l'articaïne, de la procaïne et/ou de la tétracaïne.

11. Produit selon l'une des revendications 7 à 10, **caractérisé en ce que** la concentration en antiseptique ou en anesthésique local se situe, en termes d'efficacité physiologique, dans la plage des 0,1 à 2 % par rapport au poids sec du véhicule.

12. Produit selon l'une des revendications 2 à 11, **caractérisé par** au moins une matière auxiliaire présentant un composé antimicrobien.

13. Produit selon la revendication 12, **caractérisé en ce que** le composé antimicrobien appartient à la famille des biguanides.

14. Produit selon la revendication 13, **caractérisé en ce que** la matière auxiliaire présente du chlorhydrate de polyhexaméthylène biguanide, du dichlorhydrate de chlorhexidine, du diacétate de chlorhexidine, du D-gluconate de chlorhexidine, de l'octénidine, de la taurolidine, du chlorhydrate de cétylpyridinium et/ou des mélanges de ceux-ci.

15. Produit selon l'une des revendications 12 à 14, **caractérisé en ce que** la concentration en matières auxiliaires se situe, en termes d'efficacité antimicrobienne, dans la plage des 0,5 à 2 % par rapport au poids sec du véhicule.

16. Produit selon l'une des revendications 12 à 15, **caractérisé en ce que** le composé antimicrobien présente un composé contenant de l'argent.

17. Produit selon la revendication 16, **caractérisé en ce que** le composé contenant de l'argent comprend au moins un sel d'argent soluble, un sel d'argent peu soluble, des complexes d'argent, des composés organiques d'argent et/ou de l'argent élémentaire, de préférence du chlorure d'argent.

18. Produit selon la revendication 16 ou 17, **caractérisé en ce que** les composés contenant de l'argent présentent du nitrate d'argent, de l'acétate d'argent, du carbonate d'argent, des halogénures d'argent, de la sulfadiazine d'argent, des complexes de diamine d'argent, des complexes de dithionate d'argent, du thiocyanate d'argent, de la poudre d'argent et/ou des mélanges de ceux-ci.

19. Produit selon l'une des revendications 15 à 18, **caractérisé en ce que** la concentration en matières auxiliaires se situe, en termes d'efficacité antimicrobienne, dans la plage des 0,1 à 2 %, par rapport au poids sec du véhicule.

20. Produit selon l'une des revendications précédentes, **caractérisé en ce que** le matériau biorésorbable présente une éponge à pores fins.

21. Produit selon l'une des revendications précédentes, **caractérisé en ce que** le matériau biorésorbable présente une mousse ou une éponge présentant une épaisseur de 0,5 à 4 mm dans la direction Z (voir Fig. 11).

22. Produit selon l'une des revendications précédentes, **caractérisé en ce qu'**il peut être stérilisé par le biais d'un rayonnement de 25 kGy ou par gazage à l'oxyde d'éthylène.

23. Produit selon l'une des revendications précédentes, **caractérisé en ce que** le matériau biorésorbable est dégradable dans la zone dentaire et est intégralement résorbable dans le cas où il serait avalé par mégarde.

24. Produit selon l'une des revendications précédentes, **caractérisé en ce que** le matériau biorésorbable est au moins partiellement réticulé afin d'en réduire la biodégradabilité.

25. Produit selon l'une des revendications précédentes, **caractérisé en ce que** le matériau biorésorbable peut être réticulé par des acides carboxyliques, des chlorures d'acide carboxylique, des aldéhydes d'acide carboxylique, des époxydes et/ou des aldéhydes bifonctionnels ou multifonctionnels.

26. Produit selon l'une des revendications précédentes, **caractérisé en ce que** le matériau biorésorbable est modifiable, en matière de biodégradabilité, au moyen de ponts clivables ou par exposition à des rayons γ ou β.

27. Produit selon la revendication 26, **caractérisé en ce qu'**on peut employer, comme ponts de réticulation clivables, du méthacrylate d'hydroxyéthyle.

28. Produit selon l'une des revendications précédentes, **caractérisé en ce que** le matériau biorésorbable peut être employé pour la cicatrisation des plaies et/ou la désinfection.

29. Produit selon l'une des revendications précédentes, **caractérisé en ce que** le matériau biorésorbable peut être utilisé comme tampon entre un dentier ou un appareil dentaire et la muqueuse buccale au niveau d'une plaie ou de la gencive.

30. Produit selon la revendication 1, **caractérisé en ce que** le matériau biorésorbable peut être utilisé comme adhésif pour les prothèses totales ou partielles.

31. Produit selon l'une des revendications précédentes, **caractérisé en ce qu'**il peut être utilisé pour l'hémostase dans le cas de saignements dans la zone dentaire, y compris chez les personnes se trouvant sous l'influence d'anticoagulants tels que le Marcumar, moyennant un ajout de chitosane.

32. Produit selon l'une des revendications précédentes, **caractérisé par** une adhésivité de 1 Newton ou plus.
